# EUROPEAN PATENT SPECIFICATION

(11) **EP 3 565 458 B1**
(45) Date of publication and mention of the grant of the patent: **02.11.2022**
(21) Application number: 18712016.7
(22) Date of filing: 28.02.2018
(51) Int. Cl.: A61B 5/00, A61B 90/00

(54) **ELASTOMERIC MOISTURE BARRIER FOR FORCE SENSOR**
ELASTOMERISCHE FEUCHTIGKEITSBARRIERE FÜR KRAFTSENSOREN
BARRIÈRE ELASTOMÉRIQUE POUR LA PROTECTION DE CAPTEUR DE FORCE

(30) Priority: 28.02.2017 US 201762464900 P
(43) Date of publication of application: 13.11.2019
(73) Proprietor: St. Jude Medical International Holding S.à r.l., 2449 Luxembourg (LU)
(72) Inventor: GUO, Xiaoping, Eden Prairie, Minnesota 55347 (US); HOLMBERG, James, Champlin, Minnesota 55316 (US)
(74) Representative: Kramer Barske Schmidtchen Patentanwälte PartG mbB
(86) International application number: PCT/IB2018/051292
(87) International publication number: WO 2018/158709

(56) References cited:
- US-A1- 2008 294 144
- US-A1- 2011 087 112
- US-A1- 2011 270 046
- US-A1- 2012 172 840
- US-A1- 2013 197 481
- US-B2- 8 374 670
- SIU CHUN MICHAEL HO ET AL: "FBG Sensor for Contact Level Monitoring and Prediction of Perforation in Cardiac Ablation", SENSORS, vol. 12, no. 1, 17 January 2012 (2012-01-17), pages 1002-1013, XP055479058, DOI: 10.3390/s120101002
- A. Rostami ET AL: "Microsphere and Fiber Optics based Optical Sensors" In: "Optical Sensors - New Developments and Practical Applications", 19 March 2014 (2014-03-19), InTech, XP055479182, ISBN: 978-953-51-1233-4 DOI: 10.5772/57465, figures 12,15
- Plastic Today Staff In Medical: "Tie-layer resin promotes bonding in medical polymers", Plastics Today, 15 September 2015 (2015-09-15), pages 1-5, XP055479209, Retrieved from the Internet: URL:https://www.plasticstoday.com/medical/ tie-layer-resin-promotes-bonding-medical-p olymers/20729378823261 [retrieved on 2018-05-29]
- Putnamplastics: "Tri-Layer extrusion", , 10 June 2016 (2016-06-10), XP055479202, Retrieved from the Internet: URL:http://web.archive.org/web/20160610062 816/http://www.putnamplastics.com/extrusio ns/tri-layer-extrusions [retrieved on 2018-05-29]

## Description

### BACKGROUND

### a. Field

This disclosure relates to an elastomeric moisture barrier for a force sensor. b. Background Art

In "FBG Sensor for Contact Level Monitoring and Prediction of Perforation in Cardiac Ablation" by Siu Chun, Michael Ho et al, a force sensor is disclosed. US 8,374,670 B2 discloses a catheter having a force sensing tip. US 2008/0294144 A1 discloses a tough sensing catheter.

For many years, exploration and treatment of various organs or vessels has been possible using catheter-based diagnostic and treatment systems. Such catheters are introduced through a vessel leading to the cavity of the organ to be explored or treated or alternatively can be introduced directly through an incision made in the wall of the organ. In this manner, the patient avoids the trauma and extended recuperation times typically associated with open surgical procedures.

To provide effective diagnosis or therapy, it is frequently necessary to first map the zone to be treated with great precision. One drawback of such previously known mapping systems is that they rely on manual feedback of the catheter and/or impedance measurements to determine when the catheter is properly positioned in the vessel or organ. Those systems do not measure contact forces with the vessel or organ wall or detect contact forces applied by the catheter against the organ or vessel wall that can modify the true wall location. Instead, previously known mapping methods are time-consuming, dependent upon the skill of the clinician, and cannot compensate for artifacts created by excessive contact forces.

Treatment can be employed to the zone to be treated via the same or a different catheter. Depending upon the specific treatment to be applied to the vessel or organ, the catheter may comprise any of a number of end effectors, such as but not limited to RF ablation electrodes, rotary or scissor action cutting heads, laser ablation system, injection or sewing needles, fluid conveyance systems, forceps, manipulators, mapping electrodes, endoscopic vision systems and therapeutic delivery systems such as genetic impregnation devices.

The effectiveness of such end effectors often depends on having the end effector in contact with the tissue of the wall of the organ or vessel. Many previously-known treatment systems include expandable baskets or hooks that stabilize the distal extremity of the catheter in contact with the tissue. Such arrangements, however, can be inherently imprecise due to the motion of the organ or vessel. Moreover, the previously-known systems do not provide the ability to sense the load applied to the distal extremity of the catheter by movement of the tissue wall.

For example, in the case of a cardiac ablation system, at one extreme the creation of a gap between the end effector of the treatment system and the tissue wall can render the treatment ineffective, and inadequately ablate the tissue zone. At the other extreme, if the end effector of the catheter contacts the tissue wall with excessive force, inadvertent puncturing of the tissue resulting in cardiac tamponade can occur.

### BRIEF SUMMARY

The invention is defined by appended claim 1. Preferred embodiments are defined by the dependent claims. embodiments of the present disclosure include a medical device. The medical device includes an elongate shaft extending along a shaft longitudinal axis, which includes a shaft proximal end and a shaft distal end. A force sensor is disposed on the shaft proximal end and includes a sensor proximal end and a sensor distal end. A tip is disposed on the sensor distal end. A tubular moisture barrier defines a barrier lumen, wherein the tubular moisture barrier extends along the shaft longitudinal axis between the shaft distal end and the tip, and wherein the force sensor is disposed within the barrier lumen.

Various embodiments of the present disclosure include a medical device. The medical device includes an elongate shaft that extends along a shaft longitudinal axis, which includes a shaft proximal end and a shaft distal end. A force sensor is disposed on the shaft distal end and includes a sensor proximal end and a sensor distal end. A tip disposed on the sensor distal end. A tubular moisture barrier extends along the shaft longitudinal axis between the shaft distal end and the tip and defines a barrier lumen, the tubular moisture barrier including an inner layer and an outer layer, the outer layer coaxially bonded with the inner layer, wherein the force sensor is disposed within the barrier lumen.

Various embodiments of the present disclosure include a medical device. The medical device includes an elongate shaft extending along a shaft longitudinal axis, which includes a shaft proximal end and a shaft distal end. A force sensor is disposed on the shaft distal end and includes a sensor proximal end and a sensor distal end. A tip is disposed on the sensor distal end, the tip including a tip proximal end that defines a recessed ledge that circumferentially extends around an outer proximal surface of the tip proximal end. A tubular moisture barrier extends along the shaft longitudinal axis between the shaft distal end and the tip and defines a barrier lumen, the tubular moisture barrier including an inner layer and an outer layer, the outer layer coaxially bonded with the inner layer, wherein the force sensor is disposed within the barrier lumen. A ring of polar polymer can be disposed in the recessed ledge, wherein a distal end of the tubular moisture barrier is bonded with the ring of polar polymer.

### BRIEF DESCRIPTION OF THE DRAWINGS

FIG. 1 is a block diagram of a force sensing system, in accordance with embodiments of the present disclosure.
FIG. 2 is a partial cutaway view of a distal portion of a catheter assembly having a fiber optic force sensing assembly, in accordance with embodiments of the present disclosure.
FIG. 3 is an enlarged perspective view of a fiber optic force sensing assembly, in accordance with embodiments of the present disclosure.
FIG. 4A depicts a cross-sectional side view of a medical device that includes a force sensor and a tubular moisture barrier, in accordance with embodiments of the present disclosure.
FIG. 4B depicts an enlarged cross-sectional side view of the tubular moisture barrier 174' depicted in FIG. 4A with an inner layer, outer layer, and middle layer, in accordance with embodiments of the present disclosure.
FIG. 4C depicts an enlarged cross-sectional side view of the tubular moisture barrier depicted in FIG. 4A with an inner layer and outer layer, in accordance with embodiments of the present disclosure.
Fig. 5 depicts a side view of a medical device that includes a force sensor and a tubular moisture barrier, in accordance with embodiments of the present disclosure.

### DETAILED DESCRIPTION

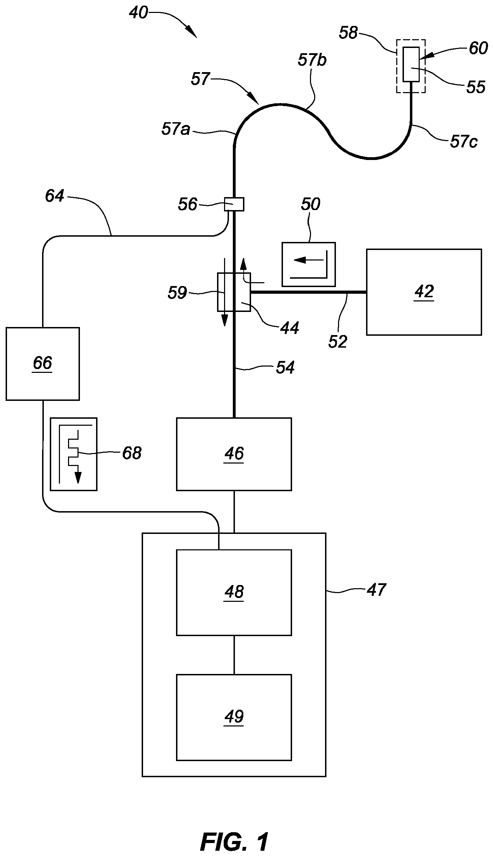

FIG. 1 is a block diagram of a force sensing system, in accordance with embodiments of the present disclosure. The sensing system 40 can comprise an electromagnetic source 42, a coupler 44, a receiver 46, an operator console 47 operatively coupled with a microprocessor 48 and a computer-readable storage device 49. The electromagnetic source 42 outputs a transmitted radiation 50 of electromagnetic radiation that is substantially steady state in nature, such as a laser or a broadband light source. A transmission line 52 such as a fiber optic cable carries the transmitted radiation 50 to the coupler 44, which directs the transmitted radiation 50 through a transmitting / receiving line 54 and through a fiber optic element, not depicted, contained within a flexible, elongate catheter assembly 57 to a fiber optic sensing element 60. The fiber optic element of the catheter assembly 57 and transmitting / receiving line 54 can be coupled through a connector 56, as depicted in FIG. 1.

The catheter assembly 57 can have a width and a length suitable for insertion into a bodily vessel or organ. In one embodiment, the catheter assembly 57 comprises a proximal portion 57a, a middle portion 57b and a distal portion 57c. The distal portion 57c can include an end effector 58 that houses the fiber optic sensing element 60. The catheter assembly 57 can be of a hollow construction (i.e., having a lumen) or of a non-hollow construction (i.e., no lumen), depending on the application. In various embodiments of the present disclosure, the catheter assembly 57 can include a gap 55 that is responsive to a contact force exerted on the end effector 58.

In one embodiment, although not depicted, a temperature sensor can be routed through the catheter assembly 57, with a lead line 64 that exits the connector 56. The lead line 64 can be routed to a temperature sensing module 66 that conditions the signal received from the temperature sensor 62 and converts it to a digital signal 68. The digital signal 68 can then be routed to the microprocessor 48 for processing.

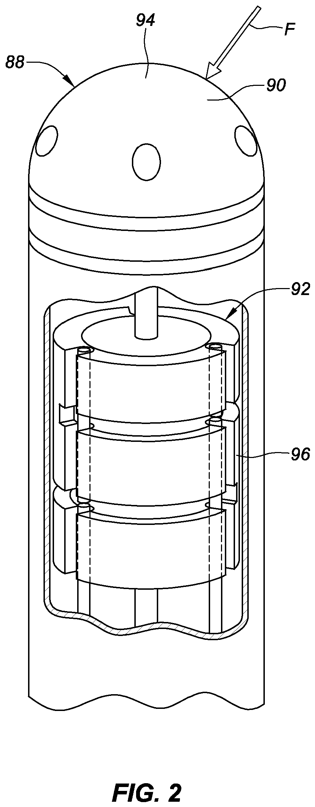

FIG. 2 is a partial cutaway view of a distal portion of a catheter assembly having a fiber optic force sensing assembly 92, in accordance with embodiments of the present disclosure. The fiber optic force sensing assembly 92 can be configured as a multisegmented structural member 96 that flexes in response to a contact force F imposed on a distal extremity 94 of end effector 88, e.g., when distal extremity 94 contacts the wall of a bodily vessel or organ. It is understood that one or more end effectors 88 of different kinds, e.g., mapping electrodes or ablation electrodes, such as are known in the art for diagnosis or treatment of a vessel or organ can be utilized with embodiments of the present disclosure. For example, the catheter assembly 57 can be configured as an electrophysiology catheter for performing cardiac mapping and ablation. In other embodiments, the catheter assembly 57 can be configured to deliver drugs or bioactive agents to a vessel or organ wall or to perform minimally invasive procedures such as transmyocardial revascularization or cryo-ablation.

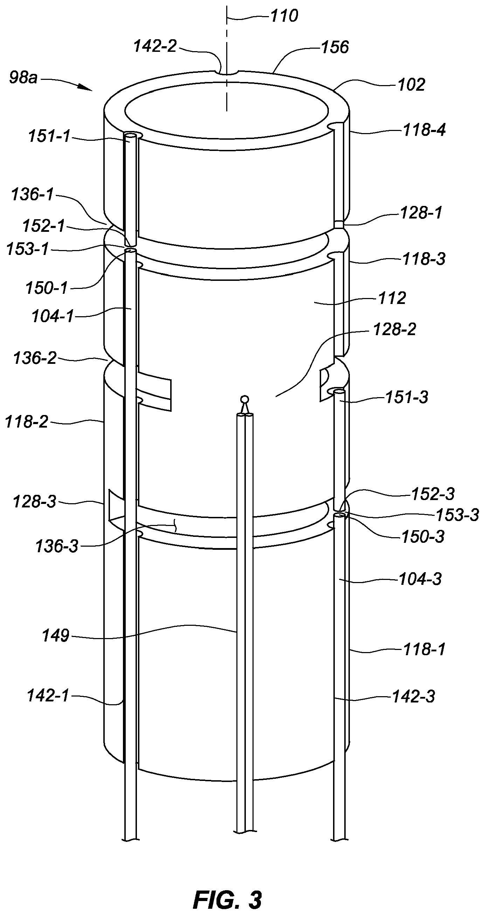

FIG. 3 is an enlarged perspective view of a fiber optic force sensing assembly 98a, in accordance with embodiments of the present disclosure, such as that discussed in relation to Fig. 2. The fiber optic force sensing assembly 98a can include a four-segment structural member 102 and a plurality of fiber optics 104-1, 104-2 (not depicted), 104-3. The structural member 102 can be formed from a metallic material, such as titanium or platinum/iridium, or a non-metallic material such as a polymer or ceramic. In some embodiments, the four-segment structural member 102 can include a hollow cylindrical tube 156 that defines a longitudinal axis 110 and includes an outer surface 112. The four-segment structural member 102 is divided into four segments, identified as a proximal segment 118-1, a first middle segment 118-2, a second middle segment 118-3 and a distal segment 118-4, hereinafter referred to in the plural as segments 118. The segments 118 are sequentially adjacent each other in a serial arrangement along the longitudinal axis 110.

In one embodiment, the segments 118 are bridged by a plurality of flexures, identified individually as flexures 128-1, 128-2 and 128-3, hereinafter referred to in the plural as flexures 128, thus defining a plurality of flexural axes. In one embodiment, adjacent members of the segments 116 define a plurality of slots, distal slot 136-1, middle slot 136-2, and proximal slot 136-3, hereinafter referred to in the plural as slots 136. In some embodiments, the distal slot can be used as an adhesive channel to attach the fiber optics 104-1, 104-2 (not depicted), 104-3.

The four-segment structural member 102 can include a plurality of grooves 142-1, 142-2, 142-3, hereinafter referred to in the plural as grooves 142, formed on the outer surface 112. The grooves 142 can be spaced rotationally equidistant (e.g., spaced 120° apart for a 3-fiber system) about the longitudinal axis 110 and can be oriented in a substantially axial direction along the four-segment structural member 102. However, in some embodiments, the grooves 142 may not be equidistant.

The fiber optics 104-1, 104-2 (not depicted), 104-3, hereinafter referred to in the plural as fiber optics 104, are disposed in the grooves 142 and can be affixed thereto with a potting (e.g., epoxy) such that their respective distal ends 150-1, 150-2 (not depicted), 150-3 terminate in the slots 136 or adjacent to the slots 136.

In some embodiments, reflecting members 151-1, 151-2 (not depicted), 151-3 each having a respective proximal end 152-1, 152-2 (not depicted), 152-3 are arranged so that the proximal ends 152 are axially in line with and distal to the distal ends 150-1, 150-2, 150-3 of each of the fiber optics 104. Each of the reflecting members 151 is paired and substantially aligned with the distal end 150 of a corresponding one of the plurality of fiber optics 104. A plurality of gaps 153-1, 153-2 (not depicted), 153-3 are defined, one between each distal end 150 of the respective fiber optic 104 and the proximal end 152 of the reflecting member 151.

The proximal ends 152 of the reflecting members 151 can be made highly reflective. The distal ends 150 of the fiber optics 104, on the other hand, can be made only partially reflective to establish the Fabry-Perot effect. When electromagnetic radiation is transmitted through the fiber optics 104, the interaction between the highly reflective proximal ends 152 of the reflecting members 151 and the partially reflective distal ends of the fiber optics 104 creates interreflections therebetween, thus establishing an interference pattern having a frequency which depends on the dimension of the gap 153. The resulting modulated waveform 74a is transmitted back through the fiber optics 104 as explained in the discussions attendant FIGS. 1 and 2.

In another embodiment, the distal ends 150 of the fiber optics 104 are not treated with the semi-reflective coating, and in fact can be treated with an anti-reflective coating (not depicted). Such an arrangement can enhance or optimize the intensity of reflected radiation that is returned to the receiver 46 via the fiber optic 53 (FIG. 1). The size of the gap 153 can be inferred from intensity of the reflected light returned as detected by the receiver 46. The intensity of the reflected light collected by a given fiber optic 104 can vary with the distance between the distal end 150 and the highly reflective proximal end 152 of the reflecting member 151.

In an example, when the optical force sensing assembly 92 (FIG. 2) is deflected in response to the force (F) being imposed on the end effector 88, the incident light rays from the optical fibers would be reflected differently from the proximal ends 152 of the reflecting members 151. Per the principle of Fabry-Perot optical interferometry, the resultant reflected light rays would produce different optical interferograms, which can be mathematically analyzed to derive the information on the external forces imposed on the sensor body or the distal ablation tip. However, the intensity of reflected radiation that is returned to the receiver 46 can vary in the presence of foreign substances that interfere with the gap 153. Obviously, the mirror gaps within the sensor body should be free of any foreign substances, including moisture that may have migrated through polymeric materials of the catheter. Otherwise, such a foreign substance may change the incident and reflected light rays, and thus optical interferogram of the force sensor, making the detection of the external forces falsified or full of noises. For example, if moisture or particulate matter enters the gaps 153, this can alter the intensity of light returned. Thus, any calculations made with respect to external forces, based on the intensity of reflected radiation returned to the receiver can be skewed. Typically, the end effector 88 may stop reporting a force because an optical signal strength is too low (e.g., signal to noise ratio is too low).

FIG. 3 further depicts a thermocouple 149 that is routed along the exterior of the structural member 102 and arranged so that the temperature sensing junction of the thermocouple 149 is in contact with the outside surface of flexure portion 128-2. In other embodiments, the temperature sensor can be routed along the interior of the structural member 102 and placed in sensing contact with the interior surfaces of the flexure portions. By sensing a temperature change of each flexure 128, a thermally-induced change to the dimensions of each flexure can be inferred and used to compensate for thermal expansion of the flexures in the calculation of a size of the gap 153.

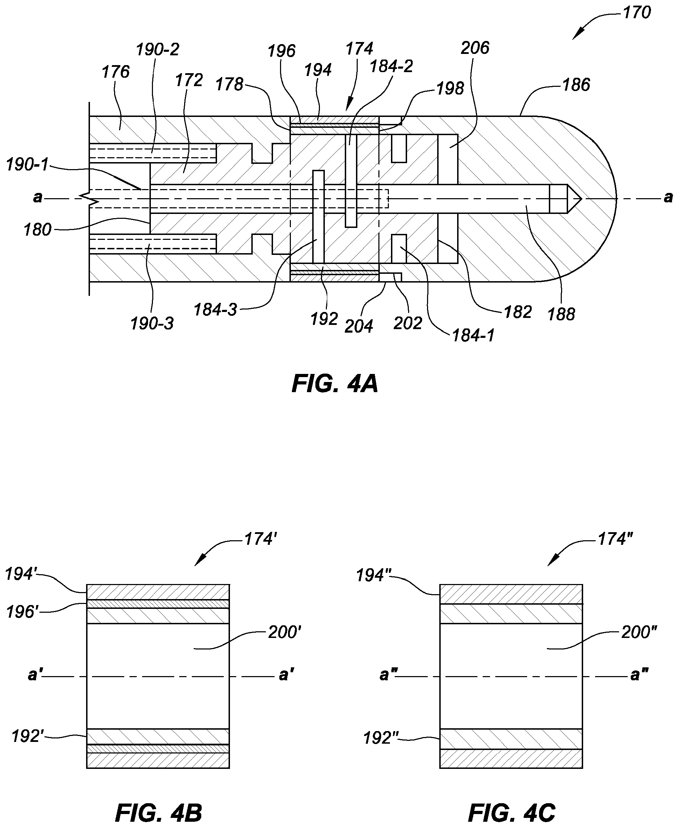

FIG. 4A depicts a cross-sectional side view of a medical device 170 that includes a force sensor 172 and a tubular moisture barrier 174, in accordance with the inventoin. The medical device 170 includes an elongate shaft 176 that extends along a shaft longitudinal axis aa, which can be formed from a polymer material. The elongate shaft 176 includes a shaft proximal end, not depicted, and a shaft distal end 178. The force sensor 172 is disposed on the shaft distal end 178 and includes a sensor proximal end 180 and a sensor distal end 182 extends along the shaft longitudinal axis aa. The distal end of the elongate shaft 176 can be connected with a proximal end of the force sensor 172 via adhesive bonding and/or thermal fusion of the shaft's polymer material. In some embodiments, the force sensor 172 can be an optical force sensing assembly, as discussed in relation to FIGS. 1 to 3. In some embodiments, the force sensor 172 can be another type of force sensor and may not necessarily include the same features as an optical force sensing assembly, as discussed herein. Furthermore, some embodiments of the present disclosure, that are not part of the invention, may include a different type of sensor in lieu of the force sensor 172. For example, embodiments of the present disclosure can include a different type of sensor, such as a position sensor, diagnostic sensor, etc.

As depicted in Fig. 4A, the force sensor 172 can define a plurality of slots, including a distal slot 184-1, a middle slot 184-2, and a proximal slot 184-3, which can allow for the force sensor 172 to deflect under application of a force to a tip 186 of the medical device 170, which can be disposed on the sensor distal end 182.

The tip 186 is formed from a metal (e.g., platinum or platinum alloy) and extends along the shaft longitudinal axis aa. The tip can define a sensor lumen 206, in which a distal end of the force sensor 172 can be inserted. The tip 186 can be permanently welded to a distal portion of the force sensor 172. The tip 186 can define an irrigation lumen 188, which can extend through a portion of the tip 186. In some embodiments, an irrigation tube can pass through an irrigation lumen that passes through a center of the force sensor 172. The irrigation tube can extend proximally from the tip 186 through the force sensor 172, and through the elongate shaft 176.

As previously discussed, the force sensor 172 can include a plurality of fiber optic elements 190-1, 190-2, 190-3, hereinafter referred to in the plural as fiber optic elements 190. A distal end of each one of the fiber optic elements 190 can be disposed in each one of the slots 184. In some embodiments, a distal end of the fiber optic elements 190-1, 190-2 can be disposed in the middle slot 184-2 and the distal end of the fiber optic element 190-3 can be disposed in the proximal slot 184-3. As previously discussed, the distal slot 184-1 can be used as an adhesive channel. Each fiber optic element 190 can include a reflecting member, as previously discussed herein, but not depicted in Fig. 4A. As discussed herein, depending on the amount of deflection of the force sensor 172, the slots can axially expand or contract, causing a varying size of gap between a distal end of each one of the fiber optic elements 190 and corresponding reflecting member, thus resulting in different optical interferograms. However, as previously discussed, if foreign substances (e.g., moisture) are present in the slots 184 and interfere with the gaps between the fiber optic elements 190 and the corresponding reflecting members, the incident and reflected light rays can be changed making the detection of the external forces falsified or full of noises. As a result, the force sensor 172 may stop reporting a force because an optical signal strength is too low (e.g., signal to noise ratio is too low), as previously discussed.

To prevent moisture migration from the irrigation tube, the irrigation tube can pass through the irrigation lumen and can be sealed (e.g., via a potting adhesive) at proximal and distal ends of the irrigation lumen defined by the force sensor 172 to prevent any potential moisture migration into the gaps between the fiber optic elements 190-1, 190-2, 190-3 and reflecting members, as further discussed herein. In some embodiments, the distal end of the irrigation lumen can be sealed via an o-ring disposed between the irrigation tube and the tip 186. Therefore, axial moisture migration from the irrigation tube or any gaps between the irrigation tube and the center lumen of the force sensor 172 into the distal slot 184-1, the middle slot 184-2, and/or the proximal slot 184-3 can be prevented.

Additionally, embodiments of the present disclosure include a tubular moisture barrier 174 that is disposed about the force sensor 172. According to the invention, the tubular moisture barrier 174 defines a barrier lumen in which the force sensor 172 is disposed. For example, the tubular moisture barrier 174 can be a hollow cylinder, which can have a circular cross-section. However, in some embodiments, the tubular moisture barrier 174 can have a square, triangular, elliptical, parabolic, polygonal or other shaped cross-section, dependent of geometry of the force sensor 172. As depicted in Fig. 4A, the tubular moisture barrier extends along the shaft longitudinal axis between the shaft distal end 178 and the tip 186.

According to the invention, , the tubular moisture barrier 174 is formed from a different material than a material that forms the elongate shaft 176. In general, polymer materials that are used for building catheter shafts can include nylons, poly(ether block amide) (PEBA) copolymers (namely commercially-available Pebax^{®} family block copolymer resin materials), thermoplastic polyurethanes, and relevant thermoplastic elastomer compounds. Typically, these shaft materials are hygroscopic, and have poor moisture barrier properties due to their inherent chemical polarity or water affinity. They may not be directly used between the elongate shaft 176 and the tip 186 for the purpose of preventing moisture from radial migration into the gaps between the fiber optic elements 190 and the reflecting members.

The tubular moisture barrier 174 includes two or more layers in some embodiments, which radially encapsulate the force sensor 172. In some embodiments, the tubular moisture barrier 174 can include two layers, three layers, four layers, etc. According to the invention, the tubular moisture barrier includes an inner layer 192 and an outer layer 194. The tubular moisture barrier 174 is connected to the shaft distal end 178, a tip proximal end 198 of the tip 186, and/or can be radially affixed to the force sensor 172 via adhesive bonding and/or thermal fusion with the tubular moisture barrier 174. As such, the tubular moisture barrier 174 can seamlessly encapsulate the middle portion of the force sensor 172, such that gaps between the fiber optic elements 190 and the reflecting members are completely enclosed by the tubular moisture barrier 174, the force sensor 172 body, and the tip 186 structures. However, in some embodiments, an air gap can exist between the inner layer and outer surface of the force sensor 172. In some embodiments, the air gap can provide room for the force sensor 172 to deflect under application of a force to the tip 186.

According to the above discussions, it is understood that in addition to its moisture barrier performance, the tubular moisture barrier 174 discussed herein exhibits rubber-like or elastomeric flexibility or elasticity such that it minimizes any interferences with deflecting motions of the force sensor 172. Moreover, the tubular moisture barrier 174 can have a sufficiently high thermal endurance within a clinical bio-environment for intended ablation procedures. In other words, one or more polymer materials used to construct the tubular moisture barrier 174 should have good thermo-mechanical integrity. This can require that the materials have sufficiently high critical thermal transitions (defined as melting temperatures for semicrystalline polymers and glass transition temperatures for amorphous polymers), which are greater than 60 °C. Also, the relevant polymer materials selected for construction of the tubular moisture barrier 174 can be thermoplastic elastomeric materials that have good melt processability, allowing for mono-extrusion and/or coextrusion, thermal lamination layer-by-layer, or reflow to catheter shaft materials (e.g., Pebax^{®}) and a thermoplastic polyurethane (TPU). Additionally, the moisture barrier tube should have adhesive bondability with the material that forms the elongate shaft 176 and materials that can form the tip 186 (e.g., metallic materials including titanium, stainless steel, and platinum).

A single polymer material may not be able to meet all the above requirements for the tubular moisture barrier. First, there can be many factors that may affect moisture permeation through a given polymer material, including crystallizability, chemical inertness to water or hydrophobicity, lack of additives and inorganic fillers, etc. The polymers with fine crystallinity and high crystallinity are likely to have good moisture barrier performances, and a typical example includes a moisture barrier material such as poly(vinylidene dichloride) (PVDC). The polymers with good crystallizability and high hydrophobicity can exhibit excellent moisture barrier performances, and typical package materials showing excellent moisture barrier behaviors are polyolefinic materials, including polyethylene homopolymers and copolymers, polypropylene homopolymers and copolymers, etc. In contrast, polar polymeric materials generally have poor moisture barrier properties, but have good chemical affinity allowing for adhesive bondability. In view of common moisture-barrier polymer materials (including PVDC and polyolefins), PVDC has poor melt processability and lacks mechanical flexibility, while polyolefins have poor adhesive bondability. Therefore, to meet the performance attributes for the barrier tube, different polymer materials are used.

It is noted that olefinic polymers generally have good moisture barrier performances, thanks to their inherent hydrophobicity and crystallizability. There are many olefinic polymers that are typically melt-processable and exhibit elastomeric flexibility. These olefinic polymers may include, linear low density polyethylenes (LLDPE), low density polyethylenes (LDPE), very low density polyethylenes (VLDPE), olefinic block copolymers (OBC), thermoplastic olefinic blends (TPO), thermoplastic vulcanizates (TPV), etc. To make good use of high adhesive bondability, typical shaft materials, including PEBA's and TPU's having high mechanical flexibility (or low durometers), are used. To integrate these two different types of polymer materials (non-polar and polar) into a layered moisture-barrier tube, a middle layer (e.g., tie layer) can be introduced in between the non-polar layer and the polar layer. The middle layer can be composed of functionalized polyolefin materials with limited polarities. Highly flexible polyolefin polymers with polar functional groups including maleic anhydride and other polar co-monomers (including vinyl acetate, acrylic acid, acrylate, carboxylic acid, carboxylic ester, epoxy, etc.) can provide limited chemical affinities, allowing for melt bondability with both non-polar polyolefin polymers and polar polymers like PEBA's and TPU's via tube coextrusion or layer-to-layer thermal lamination or reflow.

In some embodiments, the tip proximal end 198 can define a proximal recessed ledge 202 that circumferentially extends around an outer proximal surface of the tip proximal end 198. To provide a compatible surface for the distal end of the moisture barrier layer to connect with the tip proximal end 198, a ring of tie polymer 204 (e.g., Pebax^{®}, polar polymer) can be disposed in the recessed ledge 202. In some embodiments, the ring of tie polymer 204 (e.g., polar polymer) can have a thickness that is approximately the same as the outer layer 194, thus providing a surface that is connected to the tip 186 to which the outer layer 194 can be bonded. In some embodiments, the ring of polar polymer 204 can be of a greater thickness than the outer layer 194, providing a surface to which a one or more of the layers 192, 194, 196 can be bonded.

In some embodiments, as further discussed herein, the outer layer 194 of the tubular moisture barrier 174 can be a polar layer, which can be bonded to the ring of polar polymer 204. In some embodiments, the outer layer 194 of the tubular moisture barrier 174 can be a non-polar layer, which can be bonded to the ring of polar polymer 204 via adhesive bonding when the ends of the moisture barrier tube 174 are surface activated via common chemical or physical treatment like chemical etching, plasma etching, etc.

Yet, in some embodiments that are not part of the invention, , as further discussed herein, the inner layer 192 and the outer layer 194 can be formed from the same or different polar, elastomeric polymers having the melting temperatures lower than that of a middle layer formed from a nonpolar, highly elastomeric polymer, or preferably, a nonpolar, tie polymer grafted with polar functional groups including maleic anhydride, acrylic, acrylate, carboxylic acid or ester, epoxy and etc. In some embodiments, the polymer that forms the inner layer and outer layer can have melting temperatures that are lower than the melting temperature of the middle layer. In some embodiments, the melting temperature of the polymer that forms the inner layer and outer layer can have melting temperatures that are lower than the melting temperature of the middle layer by a temperature in a range from 1 degree Celsius to 100 degrees Celsius. In some embodiments, the melting temperature of the polymer that forms the inner layer and outer layer can have melting temperatures that are lower than the melting temperature of the middle layer by a temperature in a range from 5 degrees Celsius to 50 degrees Celsius. Upon thermal bonding to the distal end of catheter shaft formed of polar polymers such as poly(ether-b-amide) copolymers, thermoplastic polyurethanes, poly(ether-b-ester) copolymers and combinations thereof, the inner layer 192 and outer layer 194 of the tubular moisture barrier 174 would tend to flow and fully encapsulate the middle layer 196, thus creating the melt full of polar polymers at the interfacial region of thermal bonding that promotes the adherence between the tubular moisture barrier and the catheter shaft. For example, the inner and outer layers can be coaxially bonded at their proximal and distal ends, thus fully encapsulating the middle layer.

FIG. 4B depicts an enlarged cross-sectional side view of the tubular moisture barrier 174' depicted in FIG. 4A with an inner layer 192', outer layer 194', and middle layer 196', in accordance with embodiments of the present disclosure. As depicted, the tubular moisture barrier 174' can include an inner layer 192', an outer layer 194', and a middle layer 196', wherein the outer layer 194' is a moisture barrier layer. In some embodiments, the tubular moisture barrier 174' can extend along the shaft longitudinal axis a'a' and can define a barrier lumen 200'. In some embodiments, the inner layer 192' can be formed from a polar polymer, which can allow for a good chemical affinity for adhesive bondability to an outer surface of the force sensor 172', the distal end of the shaft, and/or the tip proximal end 198'. The inner layer 192', formed from the polar polymer can be affixed to an outer surface of the force sensor 172' via at least one of adhesive bonding, thermal fusion, and reflow. In some embodiments, the outer layer 194' can be formed from a non-polar polymer, thus providing for a moisture-barrier, preventing moisture from permeating through the outer layer 194' and reaching the slots 184. As previously discussed, in order to integrate these two different types of polymer materials (non-polar and polar) into a layered moisture-barrier tube, a middle layer 196' can be introduced in between the non-polar layer and the polar layer. In an example, the middle layer 196' can be a polymer tie layer, serving to chemically bond the outer layer 194' and the inner layer 192'.

In some embodiments, the tubular moisture barrier 174' can include a polar inner layer 192', a polar middle layer 196', and a non-polar outer layer 194'. In some embodiments, the inner layer 192' can include a polar thermoplastic elastomer (TPE) material. The polar TPE material that forms the inner layer 192' can have a hardness on the Shore scale in a range from 20D to 40D. In some embodiments, the polar TPE material that forms the inner layer 192' can be a highly flexible poly(ether block amide) (PEBA) copolymer material. In some embodiments, the polar TPE material that forms the inner layer 192' can be a highly flexible TPU material.

The outer layer 194' can include a non-polar TPE material. The non-polar TPE material that forms the outer layer 194' can have a hardness on the Shore scale in a range from 30A to 40D. In some embodiments, the non-polar TPE material that forms the outer layer 194' can include a polyolefin, polyethylene, ethylene-α-olefin random copolymer, and/or ethylene-α-olefin block copolymer. In some embodiments, the non-polar TPE material that forms the outer layer 194' can include a vulcanizate material based on a polypropylene thermopolastic matrix.

The middle layer 196' can include a grafted polyolefin material with polar functional groups. The polar functional groups can include, in some embodiments, maleic anhydride, acrylic acid, acrylate, carboxylic acid, carboxylic ester, and/or epoxy. The material that forms the middle layer 196' can have a hardness on the Shore scale in a range from 30A to 40D.

In some embodiments, the tubular moisture barrier 174' can include the inner layer 192', the outer layer 194', and the middle layer 196', wherein the inner layer 192' is a moisture barrier layer. In an example, the outer layer 194' can be formed from a polar polymer, the inner layer 192' can be formed from a non-polar polymer, and the middle layer 196' can be formed from a tie layer. In some embodiments, the outer polar polymer layer can allow for good chemical affinity for adhesive bondability to the distal end of the shaft and/or the tip proximal end 198'. The polar polymer can include a TPE material, with a hardness on the shore scale in a range from 20D to 40D. In some embodiments, the polar TPE material that forms the outer layer 194' can be a highly flexible PEBA copolymer material. In some embodiments, the polar TPE material that forms the outer layer 194' can be a highly flexible TPU material.

The inner layer 192' can include a non-polar TPE material. The non-polar TPE material that forms the inner layer 192' can have a hardness on the Shore scale in a range from 30A to 40D. In some embodiments, the non-polar TPE material that forms the inner layer 192' can include a polyolefin, polyethylene, ethylene-α-olefin random copolymer, and/or ethylene-α-olefin block copolymer. In some embodiments, the non-polar TPE material that forms the inner layer 192' can include an olefinic thermoplastic vulcanizate material. In some embodiments, the non-polar TPE material that forms the inner layer 192' can include a thermoplastic vulcanizate material based on a polypropylene thermoplastic matrix.

As previously discussed, the middle layer 196' can include a grafted polyolefin material with polar functional groups. The polar functional groups can include, in some embodiments, maleic anhydride, acrylic acid, acrylate, carboxylic acid, carboxylic ester, and/or epoxy. The material that forms the middle layer 196' can have a hardness on the Shore scale in a range from 30A to 40D.

In some embodiments, the tubular moisture barrier 174' can include the inner layer 192', the outer layer 194', and the middle layer 196', wherein the middle layer 196' serves as a moisture barrier layer. In an example, that is not part of the invention, the outer layer 194' and inner layer 192' can be formed from a polar polymer and the middle layer 196' can be formed from a non-polar polymer or preferably a nonpolar, tie polymer grafted with a polar functional group, which can include maleic anhydride, acrylic acid, acrylate, carboxylic acid, carboxylic ester, and/or epoxy. In some embodiments, that are not part of the invention, , the outer polar polymer layer and inner polar polymer layer can allow for good chemical affinity for adhesive bondability to the distal end of the shaft and/or the tip proximal end 198'. The polar polymer can include a TPE material, with a hardness on the shore scale in a range from 20D to 40D. In some embodiments, the polar TPE material that forms the outer layer 194' and the inner layer 192' can be a highly flexible PEBA copolymer material. In some embodiments, the polar TPE material that forms the outer layer 194' and the inner layer 192' can be a highly flexible TPU material.

The middle layer 196' can include a non-polar TPE material. The non-polar TPE material grafted with some polar functional group that forms the middle inner layer 196' can have a hardness on the Shore scale in a range from 30A to 40D. In some embodiments, the non-polar TPE material that forms the middle layer 196' can include a polyolefin, polyethylene, ethylene-α-olefin random copolymer, and/or ethylene-α-olefin block copolymer grafted with polar functional groups including maleic anhydride, acrylic acid, acrylate, carboxylic acid, carboxylic ester, and/or epoxy. In some embodiments, the non-polar TPE material that forms the middle layer 196' can include an olefinic thermoplastic vulcanizate material grafted with a polar functional group, which can include maleic anhydride, acrylic acid, acrylate, carboxylic acid, carboxylic ester, and/or epoxy. In some embodiments, the non-polar TPE material that forms the middle layer 196' can include a thermoplastic vulcanizate material based on a polypropylene thermoplastic matrix grafted with a polar functional group, which can include maleic anhydride, acrylic acid, acrylate, carboxylic acid, carboxylic ester, and/or epoxy.

FIG. 4C depicts an enlarged cross-sectional side view of the tubular moisture barrier 174" depicted in FIG. 4A with an inner layer 192" and outer layer 194", in accordance with the invention. . As depicted, the tubular moisture barrier 174" can include an inner layer 192" and an outer layer 194", wherein the outer layer 194" is a moisture barrier layer. According to the invention, the tubular moisture barrier 174" extends along the shaft longitudinal axis a"a" and defines a barrier lumen 200". In some embodiments, the inner layer 192" can be formed from a polar polymer, which can allow for a good chemical affinity for adhesive bondability to an outer surface of the force sensor 172", the distal end of the shaft, and/or the tip proximal end 198". The inner layer 192", formed from the polar polymer can be affixed to an outer surface of the force sensor 172" via at least one of adhesive bonding, thermal fusion, and reflow. In some embodiments, the outer layer 194" can be formed from a non-polar polymer, thus providing for a moisture-barrier, preventing moisture from permeating through the outer layer 194" and reaching the slots 184. In some embodiments, to integrate the non-polar layer and the polar layer, the non-polar layer can be grafted with a polar functional group in some embodiments.

In some embodiments, the inner layer 192" can be formed from a polar TPE material. The polar TPE material that forms the inner layer 192" can have a hardness on the Shore scale in a range from 20D to 40D. In some embodiments, the polar TPE material that forms the inner layer 192" can be a highly flexible PEBA copolymer material. In some embodiments, the polar TPE material that forms the inner layer 192" can be a highly flexible TPU material.

The outer layer 194" can include a non-polar TPE material. The non-polar TPE material that forms the outer layer 194" can have a hardness on the Shore scale in a range from 30A to 40D. In some embodiments, the non-polar TPE material that forms the outer layer 194" can include a polyolefin, polyethylene, ethylene-α-olefin random copolymer, and/or ethylene-α-olefin block copolymer. In some embodiments, the non-polar TPE material that forms the outer layer 192" can include an olefinic thermoplastic vulcanizate material. In some embodiments, the non-polar TPE material that forms the outer layer 192" can include a thermoplastic vulcanizate material based on a polypropylene thermoplastic matrix.

In some embodiments, the tubular moisture barrier 174" can include an inner layer 192" and an outer layer 194", wherein the inner layer 192" is a moisture barrier layer. In some embodiments, the inner layer 192" can be a non-polar layer and the outer layer 194" can be a polar layer. In some embodiments, the outer layer can be formed from a polar TPE material. The polar TPE material that forms the outer layer 194" can have a hardness on the Shore scale in a range from 20D to 40D. In some embodiments, the polar TPE material that forms the outer layer 194" can be a highly flexible poly(ether block amide) copolymer material. In some embodiments, the polar TPE material that forms the outer layer 194" can be a highly flexible thermoplastic polyurethane material.

The inner layer 192" can include a non-polar TPE material. The non-polar TPE material that forms the inner layer 192" can have a hardness on the Shore scale in a range from 30A to 40D. In some embodiments, the non-polar TPE material that forms the inner layer 192" can include a polyolefin, polyethylene, ethylene-α-olefin random copolymer, and/or ethylene-α-olefin block copolymer. In some embodiments, the non-polar TPE material that forms the inner layer 192" can include an olefinic thermoplastic vulcanizate material. In some embodiments, the non-polar TPE material that forms the inner layer 192" can include a thermoplastic vulcanizate material based on a polypropylene thermoplastic matrix.

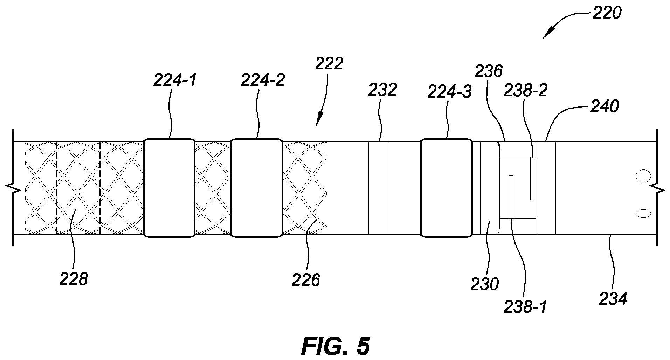

Fig. 5 depicts a side view of a medical device 220 that includes a force sensor and a tubular moisture barrier, in accordance with embodiments of the present disclosure. As depicted in FIG. 5, the medical device 220 can include an elongate shaft 222. In some embodiments, the elongate shaft 222 can be formed from a flexible PEBA copolymer, such as from the Pebax^{®} family of block copolymer materials. The PEBA copolymer can have a hardness on the Shore scale in a range from 40D to 72D, in some embodiments. The elongate shaft 222 can extend along a shaft longitudinal axis and can have one or more electrodes axially disposed thereon. For example, a first ring electrode 224-1, second ring electrode 224-2, and third ring electrode 224-3 can be disposed thereon, which can be used for mapping purposes, in some embodiments. As depicted, the elongate shaft 222, can include a braided layer 226, which longitudinally extends along a portion of the elongate shaft 222. As further depicted, the elongate shaft 222 can include a pull ring 228 disposed within the elongate shaft, to which one or more pull wires can be connected to control a deflection of the elongate shaft 222.

According to the invention, the medical device 220 includes a force sensor 230 disposed in the distal portion of the medical device 220, as discussed herein. The force sensor includes a sensor proximal end 232 and a sensor distal end (not depicted), the sensor proximal end 232 being connected to the distal end of the elongate shaft 222 and the sensor distal end being connected to a tip 234 of the medical device 220. As discussed in relation to FIG. 4A, the tip 234 is formed from a metal (e.g., platinum or platinum alloy) and can include irrigation ports.

In some embodiments, the force sensor 230 can include a deformable body having slots 238-1, 238-2, as previously discussed herein, which can include fiber optic elements that are configured to detect a force applied to the tip in response to a deflection of the force sensor 230. According to the invention, to prevent moisture from contacting the force sensor 230, a tubular moisture barrier 236 is disposed about the force sensor 230. As previously discussed herein, the tubular moisture barrier 236 includes two or more polymer layers and have a flexibility that is greater than that associated with the elongate shaft 222. In some embodiments, a proximal end of the tubular moisture barrier 236 is connected to a distal end of the elongate shaft 222 and a distal end of the tubular moisture barrier 236 is connected to a proximal end of the tip 234. In some embodiments, as discussed in relation to Fig. 4A, a proximal end of the tip 234 can define a recessed ledge in which the ring of tie polymer 240 (e.g., Pebax^{®}, polar polymer) can be disposed, which can have a hardness on the Shore scale in a range from 40D to 72D. In some embodiments, the ring of tie polymer 240 can be formed from a same material that forms the elongate shaft 222. The ring of tie polymer 240 can be adhesively bonded to the proximal end of the tip 234 (e.g., the recessed ledge) and can provide an area for bonding with the distal end of the tubular moisture barrier 236. In some embodiments, the ring of tie polymer 240 can be a polar or non-polar polymer grafted with a polar functional group. Generally, as discussed herein, the tie polymers as discussed herein can be polar or non-polar polymers grafted with a polar functional group. Furthermore, the nonpolar polymers discussed herein can include thermoplastic elastomeric materials such as polyolefins, olefinic thermoplastic elastomers, olefinic thermoplastic vulcanizates, and polymer blends of any combination thereof. Examples of the construction of the tubular moisture barrier that include inner, middle, and outer layers and inner and outer layers, according to the plurality of embodiments discussed herein are as follows.

### Example 1 - inner polar layer - middle tie layer - outer non-polar layer

Inner layer: PEBA copolymer (Arkema Pebax^{®} 3533SA01)
Middle layer: maleic anhydride-grafted ethylene-butene copolymer (Dow Amplify^{™} GR209)
Outer layer: olefinic block copolymer (Dow Infuse^{™} 9000)

In the tubular moisture barrier of Example 1, the inner layer can provide adhesive bondability with an outer surface of the force sensor, the shaft distal end, and the tip proximal end. The outer non-polar layer can act as a moisture barrier. The middle layer can act as a tie layer, allowing for melt bondability of the inner layer and the outer layer of the tubular moisture barrier via coextrusion or layer-to-layer thermal lamination (i.e. reflow) such that the layered structures are chemically bonded as an integral component without layer delamination.

In addition, individual layer thicknesses can vary. The increase in the outer layer tends to improve moisture barrier performance, while the middle layer, acting as the tie layer of the layered moisture barrier tube is kept at as minimal thickness as practical to typical tube extrusion (e.g., in a range from 0.0254 mm to 0.0127 mm (0.001" to 0.0005")). All materials used in this tri-layer moisture barrier tube structure are elastomeric (Shore 35D for Amplify^{™} GR209; Shore 35D for Pebax^{®} 3533SA01; Shore 70A (or ca. Shore 25D) for Infuse^{™} 9000); and the mechanical flexibility of the layered tube would be comparably the same or higher than that of the elongate shaft wholly made of a single Pebax 3533SA01 material. The effects of this layered moisture-barrier tube on the local deflections or probing interferograms of the force sensor body are minimal. Also, all polymer materials are semicrystalline having sufficiently high melting temperatures (ca. 115 °C for Amplify^{™} GR208; ca. 144 °C for Pebax^{®}
3533SA01; ca. 120 °C for Infuse^{™} 9000). Hence, their critical thermal transitions are all well above 60 °C. Accordingly, the tubular moisture barrier of Example 1 can withstand thermal conditions as seen in typical clinical environment such that the thermo-mechanical performances and structural integrity are well maintained.

At the ends of this tri-layer moisture barrier tube, its non-polar outer layer may be affixed onto the distal end of the shaft and the proximal end of the tip via the thin and short transient tube (e.g., the ring of tie polymer) made of the same tie-layer material (for the inner layer of the barrier tube) by reflow. Alternatively, the above affixations of the outer layer of the barrier tube with the shaft and the distal ablation tip can be achieved by adhesive bonding when the ends of the barrier tube are surface activated via common chemical or physical surface treatment like chemical etching, plasma treatment etc.

### Example 2 - inner polar layer - outer non-polar (tie) layer

Inner layer: PEBA copolymer (Arkema Pebax^{®} 3533SA01)
Outer layer: maleic anhydride-grafted ethylene-butene copolymer (Dow Amplify^{™} GR209)

The tubular moisture barrier of Example 2 is similar to Example 1, except that the outer layer consisting of the non-polar olefinic block copolymer material is eliminated. It is noted that maleic anhydride-grafted ethylene-butene copolymer still has much better moisture barrier property than typical shaft materials, such as Pebax^{®} 3533SA01, although the grafting of a polar functional group, such as maleic anhydride (Mah) onto the non-polar, high moisture-barrier ethylene-butene copolymer would compromise the original moisture barrier property to some extent. Increasing the thickness of the outer layer consisting of Mah-grafted ethylene-butene copolymer would improve the overall moisture barrier performance of this bi-layered barrier tube.

### Example 3 - inner polar layer - middle tie layer - outer non-polar layer

Inner layer: PEBA copolymer (Arkema Pebax^{®} 3533SA01)
Middle layer: ethylene-ethyl acrylate copolymer (Dow Amplify^{™} EA102)
Outer layer: olefinic block copolymer (Dow Infuse^{™} 9000)

The tubular moisture barrier of Example 3 is similar to Example 1, except that the middle layer (e.g., tie layer) is replaced by an ethylene-ethyl acrylate copolymer (EAA) material, whose durometer is ca. Shore 30D and has a melting temperature of ca. 98 °C. Due to the polarity of ethyl acrylate units, which are chemically tethered onto the non-polar ethylene units by copolymerization, the EAA material can be melt-bonded to both the olefinic block copolymer and PEBA copolymer via tube coextrusion or reflow processes. This tri-layered moisture barrier tube can exhibit similar moisture barrier performance and mechanical flexibility similar to that demonstrated by Example 1.

### Example 4 - inner polar layer - middle tie layer - outer non-polar layer

Inner layer: PEBA copolymer (Arkema Pebax^{®} 3533SA01)
Middle layer: maleic anhydride-grafted TPV (Mitsubishi Zelas^{™} MC721AP)
Outer layer: TPV (Exxon-Mobil Santoprene^{™} 8281-65MED)

The tubular moisture barrier of Example 4 employs a functionalized TPV material. The functionalized TPV material that is functionalized via grafting with a polar maleic anhydride monomer is used as the tie layer to melt-bond the inner layer consisting of the flexible PEBA copolymer and the outer layer consisting of the non-polar TPV layer, which exhibits good moisture performance.

It is known that TPV materials are part of the common thermoplastic elastomer (TPE) family. They are the dynamically-vulcanized polymeric alloys consisting mostly of fully-cured ethylene-propylene-diene rubber (EPDM) fine particles (at submicron to a few microns) encapsulated within a thermoplastic polypropylene (PP) matrix. Mechanically, PP-based TPV materials are flexible and behave like a typical vulcanized rubber. For instance, Exxon-Mobil Santoprene^{™} 8281-65MED as the outer layer of the barrier tube has Shore durometer at ca. 68A or 20D, while Mitsubishi Zelas^{™} MC721AP as the middle, tie layer has Shore durometer at ca. 75A or 25D. Hence, it is expected that the mechanical flexibility of this tri-layered barrier tube is better than the one as given in Example 1. Thanks to high hydrophobicity and crystallizability, largely originating from a PP thermoplastic matrix, this tri-layered barrier tube containing PP-based TPV materials can have sufficiently good moisture barrier performances.
This tri-layer barrier tube would also have excellent thermal endurances against clinical bio-environment with noting the facts that the two TPV materials selected (8281-65MED and MC721AP) have melting temperatures at ca. 155 and 157 °C, respectively.

### Example 5 - inner polar layer - outer non-polar (tie) layer

Inner layer: PEBA copolymer (Arkema Pebax^{®} 3533SA01)
Outer layer: maleic anhydride-grafted TPV (Mitsubishi Zelas^{™} MC721AP)

The tubular moisture barrier of Example 5 is similar to Example 4, except that the non-polar TPV material is absent. It is noted that the functionalization of a TPV material by grafting a polar maleic anhydride monomer may provide a moisture barrier, to some extent. However, such functionalization is quite limited at a very low Mah content of from 0.25 to 1.0 wt.%. Hence, the Mah-grafted TPV material generally has much better moisture barrier performances than a typical polar polymer material like Pebax^{®} 3533SA01. For convenience and economy of manufacturing and structural integrations with the catheter shaft and distal ablation tip, this bi-layered moisture barrier tube can prevent moisture migration for the intended duration of clinical procedures by increasing the thickness of the functionalized TPV outer layer.

### Example 6 - inner polar layer - middle tie layer - outer non-polar layer

Inner layer: PEBA copolymer (Arkema Pebax^{®} 3533SA01)
Middle layer: ethylene-ethyl acrylate copolymer (Dow Amplify^{™} EA102)
Outer layer: ethylene-octene copolymer (Dow Engage^{™} 8401)

The moisture barrier tube of Example 6 is similar to Example 1, except that the outer layer consisting of an olefin block copolymer is replaced by an ethylene-octene copolymer having the melting temperature of ca. 80 °C and Shore durometer at ca. 26D. As compared to Example 1, this moisture barrier tube would have relatively lower thermal endurance, which, however, is sufficiently high for the intended clinical applications.

### Example 7 - inner polar layer - middle tie layer - outer non-polar layer

Inner layer: PEBA copolymer (Arkema Pebax^{®} 3533SA01)
Middle layer: ethylene-vinyl acetate-maleic anhydride terpolymer (Arkema Orevac^{™} T9304)
Outer layer: ethylene-octene copolymer (Dow Engage^{™} 8401)

The moisture barrier tube of Example 7 is similar to Example 6, except that the middle layer (e.g., tie layer) is replaced by an ethylene-vinyl acetate-maleic anhydride terpolymer (EVA-Mah terpolymer), namely Arkema Orevac^{™} T9304, which has a melting temperature of ca. 80 °C and hardness of Shore 82A or 33D. The EVA-Mah material is a random terpolymer of ethylene, vinyl acetate and maleic anhydride, which is inherently chemically compatible with polyethylene in all proportions, namely the outer layer material. The vinyl acetate units of the middle layer material bring material flexibility and polarity, while maleic anhydride units impart some reactivity, leading to versatile adhesive properties to polar nylon-based material (namely the inner layer). With the consideration of thermal, chemical and mechanical properties of the ethylene-vinyl acetate-maleic anhydride terpolymer as the middle layer, this tri-layered moisture barrier tube would also exhibit excellent moisture-barrier properties, such as those provided by Example 6.

### Example 8 - inner non-polar (tie) layer - outer polar layer

Inner layer: maleic anhydride-grafted TPV (Mitsubishi Zelas^{™} MC721AP)
Outer layer: PEBA copolymer (Arkema Pebax^{®} 3533SA01)

The moisture barrier tube of Example 8 provides improved bondability and compatibility between the outer layer and neighboring shaft materials as compared to Example 5. Due to the moisture barrier being on the inside layer there will be a reduction in the transient moisture barrier properties compared to those provided by Example 5.

### Example 9 - inner non-polar (tie) layer - middle tie layer - outer polar layer

Inner layer: maleic anhydride-grafted TPV (Mitsubishi Zelas^{™} MC721AP)
Middle layer: maleic anhydride-grafted SEBS (Kraton FG1901)
Outer layer: PEBA copolymer (Arkema Pebax^{®} 3533SA01)

The moisture barrier tube of Example 9 prevents delamination from adjacent sections of Pebax^{®} shaft materials by allowing all layers of the tri-layer barrier tube to thermally and axially bond with the neighboring Pebax^{®} shaft material. The moisture barrier tube of Example 9 is similar to Example 8 with the addition of a tie layer to further improve bondability between the layers of the barrier tube. The integrated inner non-polar layer and middle tie layer provide intimate interfacial contact due to their limited mutual polarity arising from their respective maleic anhydride moieties, but allow for sensor deflection-induced interfacial deformability due to their inherent hydrophobicity. The middle tie layer having both styrenic and maleic anhydride functionalities improves its chemical bonding with the outer PEBA layer to impart excellent layer integrity and further prevent delamination. As a result, the barrier lumen is fully enclosed by the axially-bonded Pebax shaft material, while the barrier tube has improved compliance for deformability without compromise on layer integrity. Due to the moisture barrier being on the inner and middle layers and the barrier lumen being fully enclosed by axially-bonded Pebax shaft material, there will be a reduction in the transient moisture barrier properties compared to those provided by Example 5.

### Example 10 - inner polar layer - middle nonpolar (tie) layer- outer polar layer

Inner layer: PEBA copolymer (Arkema Pebax^{®} 3533SA01)
Middle layer: maleic anhydride-grafted, polypropylene-based TPV (Mitsubishi Zelas^{™} MC721AP)
Outer layer: PEBA copolymer (Arkema Pebax^{®} 3533SA01)

In example 10, that is not part of the claimed invention, the non-polar middle layer can comprise a maleic anhydride-grafted, PP-based TPV material, which also serves as a tie layer providing good layer-to-layer adherence to the inner and outer layers, in addition to its inherent moisture barrier performance. As compared to the inner and outer layers comprising the same PEBA copolymer, i.e. Pebax^{®} 3533 SA01, having the relatively lower melting temperature (ca. 144 degrees Celsius), the non-polar layer comprising Zelas^{™} MC721AP has the relatively higher melting temperature (ca. 157 degrees Celsius). As such, when the moisture barrier tube is disposed and thermally bonded to the distal end of catheter shaft made of a PEBA copolymer (i.e. a material from Pebax^{®} family) or a thermoplastic polyurethane material, the inner and outer layers of the barrier tube would tend to flow and fully encapsulate the middle layer of the barrier tube, and thus advantageously forms very strong thermal bonds at the tube-shaft interfacial region largely occupied by the melts of the polar polymer materials.

Embodiments are described herein of various apparatuses, systems, and/or methods. Numerous specific details are set forth to provide a thorough understanding of the overall structure, function, manufacture, and use of the embodiments as described in the specification and depicted in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," or "an embodiment", or the like, means that a particular feature, structure, or characteristic described in connection with the embodiment(s) is included in at least one embodiment. Thus, appearances of the phrases "in various embodiments," "in some embodiments," "in one embodiment," or "in an embodiment," or the like, in places throughout the specification, are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features, structures, or characteristics of one or more other embodiments without limitation given that such combination is not illogical or non-functional.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting and absolute.

All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the devices. Joinder references (e.g., affixed, attached, coupled, connected, and the like) are to be construed broadly and can include intermediate members between a connection of elements and relative movement between elements. As such, joinder references do not necessarily infer that two elements are directly connected and in fixed relationship to each other. It is intended that all matter contained in the above description or shown in the accompanying drawings shall be interpreted as illustrative only and not limiting. Changes in detail or structure can be made without departing from the scope of the disclosure as defined in the appended claims.

## Claims

1. A medical device, comprising
an elongate shaft (176; 222) extending along a shaft longitudinal axis and including a shaft proximal end and a shaft distal end (178),
a force sensor (172; 230) disposed on the shaft distal end (178) and including a sensor proximal end and a sensor distal end,
a tip (186; 234) disposed on the sensor distal end, and
a tubular moisture barrier (174; 236) defining a barrier lumen, wherein
the tubular moisture barrier extends along the shaft longitudinal axis between the shaft distal end (178) and the tip (186; 234),
the force sensor (172; 230) is disposed within the barrier lumen,
the tubular moisture barrier (174; 236) includes an inner layer (192) and an outer layer (194), the tubular moisture barrier (174; 236) is connected to a proximal end of the tip (186; 234),
the tip (186; 234) is formed from metal,
the force sensor is configured to deflect in response to a force applied to the tip,
the tubular moisture barrier is configured to deflect with the force sensor in response to the force applied to the tip,
the tubular moisture barrier (174; 236) has a greater flexibility than the elongate shaft,
the tubular moisture barrier (174; 236) is formed from a different material than a material that forms the elongate shaft, and
one of the inner layer (192) and outer layer (194) of the tubular moisture barrier (174, 236) is a PEBA copolymer or a thermoplastic polyurethane (TPU) and the other layer a non-polar polymer.

2. The medical device of claim 1, wherein the inner layer (192) is formed from a PEBA copolymer or a thermoplastic polyurethane (TPU) and the outer layer (194) is formed from a non-polar polymer, the medical device preferably further comprising a middle layer (196) disposed between the inner layer (192) and the outer layer (194), wherein the middle layer (196) is formed from a polymer tie layer.

3. The medical device of claim 1, wherein the inner layer (192) is formed from a non-polar polymer and the outer layer (194) is formed from a PEBA copolymer or a thermoplastic polyurethane (TPU), the medical device preferably further comprises a middle layer (196) disposed between the inner layer (192) and the outer layer (194), wherein the middle layer (196) is formed from a polymer tie layer.

4. The medical device of claim 1, further comprising a middle layer (196) disposed between the inner layer (192) and the outer layer (194), wherein the middle layer (196) is formed from a nonpolar polymer and the inner layer (192) and the outer layer (194) are formed from different polymers, wherein a melting temperature of the inner layer and the outer layer is lower than a melting temperature of the middle layer by a temperature in a range from 1 degree Celsius to 100 degrees Celsius.

5. The medical device of claim 4, wherein a melting temperature of the inner layer (192) and the outer layer (194) is lower than a melting temperature of the middle layer (196) by a temperature in a range from 5 degrees Celsius to 50 degrees Celsius.

6. The medical device of any one of claims 1 to 5, wherein the tubular moisture barrier (174; 236) is formed from a flexible polymer.

7. The medical device of any one of claims 1 to 6, wherein the tubular moisture barrier (174; 236) is connected to the shaft distal end.

8. The medical device of claim 1, wherein a material that forms the tubular moisture barrier is a thermoplastic material that has a thermal transition temperature of greater than 60 degrees Celsius.

9. The medical device of claim 1, wherein the outer layer (194) is coaxially bonded with the inner layer (192).

10. The medical device of claim 9, wherein the inner layer (192) is formed from a PEBA copolymer or a thermoplastic polyurethane (TPU) poly and the outer layer (194) is formed from a non-polar polymer, wherein the medical device further comprises a tie layer disposed between the inner layer and the outer layer.

11. The medical device of claim 9, wherein the inner layer (192) is formed from a non-polar polymer and the outer layer (194) is formed from a PEBA copolymer or a thermoplastic polyurethane (TPU), wherein the medical device further comprises a tie layer disposed between the non-polar polymer and the PEBA copolymer or thermoplastic polyurethane (TPU).

12. The medical device of any one of claims 1 to 11, wherein the tip (196) includes a tip proximal end that defines a recessed ledge (202) that circumferentially extends around an outer proximal surface of the tip proximal end, and
a ring (204) of tie polymer is disposed in the recessed ledge, wherein a distal end of the tubular moisture barrier is bonded with the ring of tie polymer,
wherein the outer layer (194) and the inner layer (192) are formed from polymer materials with different polarity, wherein the inner layer (192) and the outer layer (194) are bonded with the ring (204) of polar polymer.

## Patentansprüche

1. Medizinische Vorrichtung, mit
einem länglichen Schaft (176; 222), der sich entlang einer Schaftlängsachse erstreckt und ein proximales Schaftende und ein distales Schaftende (178) aufweist,
einem Kraftsensor (172; 230), der an dem distalen Schaftende (178) angeordnet ist und ein proximales Sensorende und ein distales Sensorende aufweist,
einer Spitze (186; 234), die an dem distalen Sensorende angeordnet ist, und
einer rohrförmigen Feuchtigkeitssperre (174; 236), die ein Sperrlumen definiert, bei der
die rohrförmige Feuchtigkeitssperre sich entlang der Schaftlängsachse zwischen dem distalen Schaftende (178) und der Spitze (186; 234) erstreckt,
der Kraftsensor (172; 230) innerhalb des Sperrlumens angeordnet ist,
die rohrförmige Feuchtigkeitssperre (174; 236) eine innere Schicht (192) und eine äußere Schicht (194) aufweist, und die rohrförmige Feuchtigkeitssperre (174; 236) mit einem proximalen Ende der Spitze (186; 234) verbunden ist,
die Spitze (186; 234) aus Metall ausgebildet ist,
der Kraftsensor dazu konfiguriert ist, in Antwort auf eine Kraft, die auf die Spitze aufgebracht wird, auszulenken,
die rohrförmige Feuchtigkeitssperre dazu konfiguriert ist, mit dem Kraftsensor in Antwort auf die Kraft, die auf die Spitze aufgebracht wird, auszulenken,
die rohrförmige Feuchtigkeitssperre (174; 236) eine größere Flexibilität als der längliche Schaft aufweist,
die rohrförmige Feuchtigkeitssperre (174; 236) aus einem unterschiedlichen Material als ein Material ausgebildet ist, das den länglichen Schaft ausbildet,
eine von der inneren Schicht (192) und der äußeren Schicht (194) der rohrförmigen Feuchtigkeitssperre (174, 236) ein PEBA-Copolymer oder ein thermoplastisches Polyurethan (TPU) ist, und die andere Schicht ein nicht-polares Polymer ist.

2. Medizinische Vorrichtung nach Anspruch 1, bei der die innere Schicht (192) aus einem PEBA-Copolymer oder einem thermoplastischen Polyurethan (TPU) ausgebildet ist, und
die andere Schicht (194) aus einem nicht-polaren Polymer ausgebildet ist,
die medizinische Vorrichtung bevorzugt ferner eine mittlere Schicht (196) aufweist, die zwischen der inneren Schicht (192) und der äußeren Schicht (194) angeordnet ist, bei der die mittlere Schicht (196) aus einer polymeren Bindeschicht ausgebildet ist.

3. Medizinische Vorrichtung nach Anspruch 1, bei der die innere Schicht (192) aus einem nicht-polaren Polymer ausgebildet ist, und die äußere Schicht (194) aus einem PEBA-Copolymer oder einem thermoplastischen Polyurethan (TPU) ausgebildet ist,
die medizinische Vorrichtung bevorzugt ferner eine mittlere Schicht (196) aufweist, die zwischen der inneren Schicht (192) und der äußeren Schicht (194) angeordnet ist, bei der die mittlere Schicht (196) aus einer polymeren Bindeschicht ausgebildet ist.

4. Medizinische Vorrichtung nach Anspruch 1, ferner mit einer mittleren Schicht (196), die zwischen der inneren Schicht (192) und der äußeren Schicht (194) angeordnet ist, bei der die mittlere Schicht (196) aus einem nicht-polaren Polymer ausgebildet ist und die innere Schicht (192) und die äußere Schicht (194) aus unterschiedlichen Polymeren ausgebildet sind, bei der eine Schmelztemperatur der inneren Schicht und der äußeren Schicht geringer als eine Schmelztemperatur der mittleren Schicht durch eine Temperatur in einem Bereich von 1 Grad Celsius bis 100 Grad Celsius ist.

5. Medizinische Vorrichtung nach Anspruch 4, bei der eine Schmelztemperatur der inneren Schicht (192) und der äußeren Schicht (194) geringer als eine Schmelztemperatur der mittleren Schicht (196) durch eine Temperatur in einem Bereich von 5 Grad Celsius bis 50 Grad Celsius ist.

6. Medizinische Vorrichtung nach einem der Ansprüche 1 bis 5, bei der die rohrförmige Feuchtigkeitssperre (174; 236) aus einem flexiblen Polymer ausgebildet ist.

7. Medizinische Vorrichtung nach einem der Ansprüche 1 bis 6, bei der die rohrförmige Feuchtigkeitssperre (174; 236) mit dem distalen Schaftende verbunden ist.

8. Medizinische Vorrichtung nach Anspruch 1, bei der ein Material, das die rohrförmige Feuchtigkeitssperre ausbildet, ein thermoplastisches Material ist, das eine thermische Übergangstemperatur von größer als 60 Grad Celsius aufweist.

9. Medizinische Vorrichtung nach Anspruch 1, bei der die äußere Schicht (194) koaxial mit der inneren Schicht (192) verbunden ist.

10. Medizinische Vorrichtung nach Anspruch 9, bei der die innere Schicht (192) aus einem PEBA-Copolymer oder einem thermoplastischen Polyurethan (TPU) ausgebildet ist und die äußere Schicht (194) aus einem nicht-polaren Polymer ausgebildet ist,
bei der die medizinische Vorrichtung ferner eine Bindeschicht aufweist, die zwischen der inneren Schicht und der äußeren Schicht angeordnet ist.

11. Medizinische Vorrichtung nach Anspruch 9, bei der die innere Schicht (192) aus einem nicht-polaren Polymer ausgebildet ist und die äußere Schicht (194) aus einem PEBA-Copolymer oder einem thermoplastischen Polyurethan (TPU) ausgebildet ist,
bei der die medizinische Vorrichtung ferner eine Bindeschicht aufweist, die zwischen dem nicht-polaren Polymer und dem PEBA-Copolymer oder thermoplastischen Polyurethan (TPU) angeordnet ist.

12. Medizinische Vorrichtung nach einem der Ansprüche 1 bis 11, bei der die Spitze (196) ein proximales Spitzenende aufweist, das einen ausgenommenen Absatz (202) definiert, der sich umfänglich um eine äußere proximale Oberfläche des proximalen Spitzenendes erstreckt,
ein Ring (204) aus bindendem Polymer in dem ausgenommenen Absatz angeordnet ist, bei der ein distales Ende der rohrförmigen Feuchtigkeitssperre mit dem Ring des verbundenen Polymers verbunden ist,
bei der die äußere Schicht (194) und die innere Schicht (192) aus Polymermaterialien mit unterschiedlicher Polarität ausgebildet sind, bei der die innere Schicht (192) und die äußeren Schicht (195) mit dem Ring (204) aus polarem Polymer verbunden sind.

## Revendications

1. Dispositif médical, comprenant
un arbre allongé (176; 222) s'étendant le long d'un axe longitudinal d'arbre et comprenant une extrémité proximale d'arbre et une extrémité distale d'arbre (178),
un capteur de force (172 ; 230) disposé sur l'extrémité distale d'arbre (178) et comprenant une extrémité proximale de capteur et une extrémité distale de capteur,
une pointe (186 ; 234) disposée sur l'extrémité distale de capteur, et
une barrière tubulaire contre l'humidité (174 ; 236) définissant une lumière de barrière, dans lequel
la barrière tubulaire contre l'humidité s'étend le long de l'axe longitudinal d'arbre entre l'extrémité distale d'arbre (178) et la pointe (186 ; 234),
le capteur de force (172 ; 230) est disposé à l'intérieur de la lumière de barrière,
la barrière tubulaire contre l'humidité (174 ; 236) comprend une couche interne (192) et une couche externe (194), la barrière tubulaire contre l'humidité (174 ; 236) est reliée à une extrémité proximale de la pointe (186 ; 234),
la pointe (186 ; 234) est formée à partir de métal,
le capteur de force est configuré pour dévier en réponse à une force appliquée à la pointe,
la barrière tubulaire contre l'humidité est configurée pour dévier avec le capteur de force en réponse à la force appliquée à la pointe,
la barrière tubulaire contre l'humidité (174 ; 236) a une plus grande flexibilité que l'arbre allongé,
la barrière tubulaire contre l'humidité (174; 236) est formée d'un matériau différent d'un matériau qui forme l'arbre allongé, et
une couche parmi la couche interne (192) et la couche externe (194) de la barrière tubulaire contre l'humidité (174, 236) est un copolymère PEBA ou un polyuréthane thermoplastique (TPU) et l'autre couche est un polymère non polaire.

2. Dispositif médical selon la revendication 1, dans lequel la couche interne (192) est formée d'un copolymère PEBA ou d'un polyuréthane thermoplastique (TPU) et la couche externe (194) est formée d'un polymère non polaire,
le dispositif médical comprenant de préférence en outre une couche intermédiaire (196) disposée entre la couche interne (192) et la couche externe (194), dans lequel la couche intermédiaire (196) est formée d'une couche de liaison de polymère.

3. Dispositif médical selon la revendication 1, dans lequel la couche interne (192) est formée d'un polymère non polaire et la couche externe (194) est formée d'un copolymère PEBA ou d'un polyuréthane thermoplastique (TPU),
le dispositif médical comprend de préférence en outre une couche intermédiaire (196) disposée entre la couche interne (192) et la couche externe (194), dans lequel la couche intermédiaire (196) est formée d'une couche de liaison de polymère.

4. Dispositif médical selon la revendication 1, comprenant en outre une couche intermédiaire (196) disposée entre la couche interne (192) et la couche externe (194), dans lequel la couche intermédiaire (196) est formée à partir d'un polymère non polaire et la couche interne (192) et la couche externe (194) sont formées à partir de polymères différents, dans lequel une température de fusion de la couche interne et de la couche externe est inférieure à une température de fusion de la couche intermédiaire d'une température dans une plage de 1 degré Celsius à 100 degrés Celsius.

5. Dispositif médical selon la revendication 4, dans lequel une température de fusion de la couche interne (192) et de la couche externe (194) est inférieure à une température de fusion de la couche intermédiaire (196) d'une température dans une plage de 5 degrés Celsius à 50 degrés Celsius.

6. Dispositif médical selon l'une quelconque des revendications 1 à 5, dans lequel la barrière tubulaire contre l'humidité (174 ; 236) est formée d'un polymère flexible.

7. Dispositif médical selon l'une quelconque des revendications 1 à 6, dans lequel la barrière tubulaire contre l'humidité (174 ; 236) est reliée à l'extrémité distale d'arbre.

8. Dispositif médical selon la revendication 1, dans lequel un matériau qui forme la barrière tubulaire contre l'humidité est un matériau thermoplastique qui a une température de transition thermique supérieure à 60 degrés Celsius.

9. Dispositif médical selon la revendication 1, dans lequel la couche externe (194) est liée coaxialement à la couche interne (192).

10. Dispositif médical selon la revendication 9, dans lequel la couche interne (192) est formée d'un copolymère PEBA ou d'un polyuréthane thermoplastique (TPU) et la couche externe (194) est formée d'un polymère non polaire,
dans lequel le dispositif médical comprend en outre une couche de liaison disposée entre la couche interne et la couche externe.

11. Dispositif médical selon la revendication 9, dans lequel la couche interne (192) est formée d'un polymère non polaire et la couche externe (194) est formée d'un copolymère PEBA ou d'un polyuréthane thermoplastique (TPU),
dans lequel le dispositif médical comprend en outre une couche de liaison disposée entre le polymère non polaire et le copolymère PEBA ou le polyuréthane thermoplastique (TPU).

12. Dispositif médical selon l'une quelconque des revendications 1 à 11, dans lequel la pointe (196) comprend une extrémité proximale de pointe qui définit un rebord évidé (202) qui s'étend circonférentiellement autour d'une surface proximale extérieure de l'extrémité proximale de pointe, et
un anneau (204) de polymère de liaison est disposé dans le rebord évidé, dans lequel une extrémité distale de la barrière tubulaire contre l'humidité est liée à l'anneau de polymère de liaison,
dans lequel, de préférence, la couche externe (194) et la couche interne (192) sont formées à partir de matériaux polymères de polarité différente, dans lequel la couche interne (192) et la couche externe (194) sont liées à l'anneau (204) de polymère polaire.
